(19) Europäisches Patentamt
European Patent Office
Office européen des brevets

(11) Veröffentlichungsnummer: **0 397 295**
**A2**

# (12) EUROPÄISCHE PATENTANMELDUNG

(21) Anmeldenummer: **90250119.6**

(22) Anmeldetag: **08.05.90**

(51) Int. Cl.5: **C07D 209/46, C07D 209/44, A01N 43/48**

(30) Priorität: **12.05.89 DE 3915953**

(43) Veröffentlichungstag der Anmeldung:
**14.11.90 Patentblatt 90/46**

(84) Benannte Vertragsstaaten:
**AT BE CH DE DK ES FR GB GR IT LI LU NL SE**

(71) Anmelder: **SCHERING AKTIENGESELLSCHAFT**
**Müllerstrasse 170/178**
**D-1000 Berlin 65(DE)**

(72) Erfinder: **Franke, Wilfried, Dr.**
**Spiessergasse 6B**
**D-1000 Berlin 27(DE)**
Erfinder: **Ganzer, Michael, Dr.**
**Eichborndamm 279**
**D-1000 Berlin 26(DE)**
Erfinder: **Dorfmeister, Gabriele, Dr.**
**Heiligenseestrasse 70**
**D-1000 Berlin 27(DE)**
Erfinder: **Rees, Richard, Dr.**
**Speerweg 8**
**D-1000 Berlin 28(DE)**
Erfinder: **Johann, Gerhard, Dr.**
**Hermsdorfer Damm 147**
**D-1000 Berlin 28(DE)**

(54) **2-Phenyl-perhydro-isoindol-1-one und -1-thione, Verfahren zu ihrer Herstellung und ihre Verwendung als Mittel mit herbizider Wirkung.**

(57) Die Erfindung betrifft neue 2-Phenyl-perhydro-isoindol-1-one und -1-thione der allgemeinen Formel I

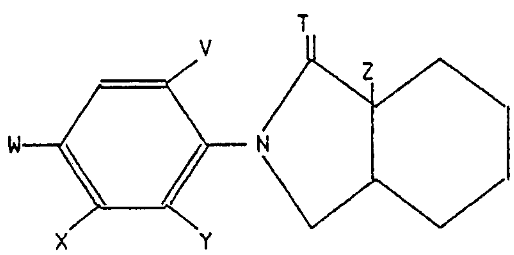

(I) ,

in der T, V, W, X, Y und Z die in der Beschreibung genannten Bedeutungen haben, Verfahren zu ihrer Herstellung und ihre Verwendung als Mittel mit herbizider Wirkung.

EP 0 397 295 A2

# 2-PHENYL-PERHYDRO-ISOINDOL-1-ONE UND -1-THIONE, VERFAHREN ZU IHRER HERSTELLUNG UND IHRE VERWENDUNG ALS MITTEL MIT HERBIZIDER WIRKUNG

Die Erfindung betrifft neue 2-Phenyl-perhydro-isoindol-1-one und -1-thione, Verfahren zu ihrer Herstellung und ihre Verwendung als Mittel mit herbizider Wirkung.

Es ist bereits bekannt, daß N-Phenyl-hexahydro-isoindol-verbindungen herbizide Eigenschaften besitzen (DE 36 18 501 A1). Häufig ist jedoch die Herbizidwirkung dieser bekannten Verbindungen nicht ausreichend, oder es treten Selektivitätsprobleme in wichtigen landwirtschaftlichen Kulturen auf.

Aufgabe der vorliegenden Erfindung ist daher die Bereitstellung von neuen Verbindungen, die diese Nachteile nicht aufweisen und in ihren biologischen Eigenschaften den bisher bekannten überlegen sind.

Es wurde nun gefunden, daß 2-Phenyl-perhydro-isoindol-1-one und -1-thione der allgemeinen Formel I

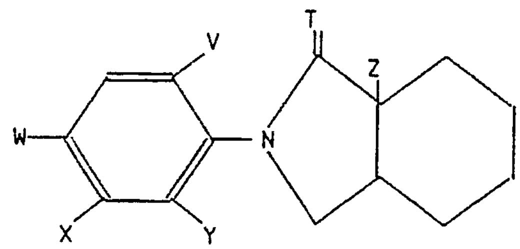

(I) ,

in der

V ein Wasserstoffatom, ein Fluoratom oder ein Chloratom,

W ein Wasserstoffatom oder ein Halogenatom,

X ein Wasserstoffatom, ein Halogenatom, einen Trihalogenmethylrest oder eine der Gruppen $-OR^1$, $-SR^1$ oder $-CO_2R^2$, oder

W und X zusammen eine Gruppierung $-O-CH_2-CO-NR^3-$, $-S-CO-NR^3-$ oder $-O-CO-NR^3-$, wobei $-O-CH_2-$, -S- oder -O- für W stehen,

$R^1$ ein Wasserstoffatom, einen $C_1$-$C_6$-Alkylrest, einen ein- oder mehrfach durch Halogen substituierten $C_1$-$C_6$-Alkylrest, einen $C_2$-$C_6$-Alkenylrest, einen ein- oder mehrfach durch Halogen substituierten $C_2$-$C_6$-Alkenylrest, einen $C_3$-$C_6$-Alkinylrest, einen ein- oder mehrfach durch Halogen substituierten $C_3$-$C_6$-Alkinylrest, einen $C_3$-$C_6$-Cycloalkylrest, einen ein- oder mehrfach durch Halogen substituierten $C_3$-$C_6$-Cycloalkylrest, einen $C_3$-$C_6$- Cycloalkyl-$C_1$-$C_3$-alkylrest, einen ein- oder mehrfach durch Halogen substituierten $C_3$-$C_6$-Cycloalkyl-$C_1$-$C_3$-alkylrest, einen Hydroxycarbonyl-$C_1$-$C_6$-alkylrest, einen ein- oder mehrfach durch Halogen substituierten Hydroxycarbonyl-$C_1$-$C_6$-alkylrest, einen $C_1$-$C_6$-Alkoxycarbonyl-$C_1$-$C_6$-alkylrest, einen ein- oder mehrfach durch Halogen substituierten $C_1$-$C_6$-Alkoxycarbonyl-$C_1$-$C_6$-alkylrest, einen $C_2$-$C_6$-Alkenyloxycarbonyl-$C_1$-$C_6$-alkylrest, einen ein- oder mehrfach durch Halogen substituierten $C_2$-$C_6$-Alkenyloxycarbonyl-$C_1$-$C_6$-alkylrest, einen $C_3$-$C_6$-Alkinyloxycarbonyl-$C_1$-$C_6$-alkylrest, einen ein- oder mehrfach durch Halogen substituierten $C_3$-$C_6$-Alkinyloxycarbonyl-$C_1$-$C_6$-alkylrest, einen $C_3$-$C_6$-Cycloalkyloxycarbonyl-$C_1$-$C_6$-alkylrest, einen ein- oder mehrfach durch Halogen substituierten $C_3$-$C_6$-Cycloalkyloxycarbonyl-$C_1$-$C_6$-alkylrest, einen $C_1$-$C_4$-Alkylsulfonylrest, einen ein- oder mehrfach durch Halogen substituierten $C_1$-$C_4$-Alkylsulfonylrest, einen Phenylsulfonylrest, einen ein- oder mehrfach, gleich oder verschieden durch Halogen, $C_1$-$C_4$-Alkyl, Halogen-$C_1$-$C_4$-alkyl, Nitro, $C_1$-$C_4$-Alkoxy oder Halogen-$C_1$-$C_4$-alkoxy substituierten Phenylsulfonylrest, einen durch einen gesättigten oder ungesättigten Heterocyclus substituierten $C_1$-$C_4$-Alkylrest, einen Phenylrest oder einen ein- oder mehrfach, gleich oder verschieden durch Halogen, $C_1$-$C_4$-Alkyl, Halogen-$C_1$-$C_4$-alkyl, Nitro, $C_1$-$C_4$-Alkoxy oder Halogen-$C_1$-$C_4$-alkoxy substituierten Phenylrest,

$R^2$ ein Wasserstoffatom, einen $C_1$-$C_6$-Alkylrest, einen ein- oder mehrfach durch Halogen substituierten $C_1$-$C_6$-Alkylrest, einen $C_2$-$C_6$-Alkenylrest, einen ein- oder mehrfach durch Halogen substituierten $C_2$-$C_6$-Alkenylrest, einen $C_3$-$C_6$-Alkinylrest, einen ein- oder mehrfach durch Halogen substituierten $C_3$-$C_6$-Alkinylrest, einen $C_3$-$C_6$-Cycloalkylrest, einen ein- oder mehrfach durch Halogen substituierten $C_3$-$C_6$-Cycloalkylrest, einen $C_3$-$C_6$-Cycloalkyl-$C_1$-$C_3$-alkylrest oder einen ein- oder mehrfach durch Halogen substituierten $C_3$-$C_6$-Cycloalkyl-$C_1$-$C_3$-alkylrest,

$R^3$ ein Wasserstoffatom, einen $C_1$-$C_6$-Alkylrest, einen ein- oder mehrfach durch Halogen substituierten $C_1$-$C_6$-Alkylrest, einen $C_2$-$C_6$-Alkenylrest, einen ein- oder mehrfach durch Halogen substituierten $C_2$-$C_6$-Alkenylrest, einen $C_3$-$C_6$-Alkinylrest, einen ein- oder mehrfach durch Halogen substituierten $C_3$-$C_6$-Alkinyl-

rest, einen $C_3$-$C_6$-Cycloalkylrest, einen ein- oder mehrfach durch Halogen substituierten $C_3$-$C_6$-Cycloalkylrest, einen $C_3$-$C_6$-Cycloalkyl-$C_1$-$C_3$-alkylrest oder einen ein- oder mehrfach durch Halogen substituierten $C_3$-$C_6$-Cycloalkyl-$C_1$-$C_3$-alkylrest,
Y ein Wasserstoffatom, ein Fluoratom, ein Chloratom oder eine Gruppe $NR^4R^5$,
$R^4$ ein Wasserstoffatom, einen $C_1$-$C_6$-Alkylrest, einen $C_2$-$C_6$-Alkenylrest oder einen $C_3$-$C_6$-Alkinylrest,
$R^5$ ein Wasserstoffatom, einen $C_1$-$C_6$-Alkylrest, einen $C_2$-$C_6$-Alkenylrest oder einen $C_3$-$C_6$-Alkinylrest,
T ein Sauerstoffatom oder ein Schwefelatom und
Z ein Wasserstoffatom, ein Chloratom oder ein Bromatom, bedeuten, eine interessante herbizide Wirkung zeigen.

Die Verbindungen der allgemeinen Formel I können gegebenenfalls in verschiedenen enantiomeren, diastereomeren oder geometrischen Formen anfallen und sind ebenfalls Gegenstand dieser Erfindung.

Die Bezeichnung "Halogen" umfaßt Fluor, Chlor, Brom und Jod.

Die Bezeichnung "Halogenalkyl" besagt, daß ein oder mehrere Wasserstoffatome des Alkylrestes durch Halogen ersetzt sind.

Unter einem gesättigten oder ungesättigten Heterocyclus sind zum Beispiel zu verstehen Tetrahydrofuran, Tetrahydrothiophen, Pyrrolidin, Piperidin, Morpholin, Pyridin oder Pyrrol.

Die erfindungsgemäßen Verbindungen der allgemeinen Formel I lassen sich herstellen, indem man

A) falls T für Sauerstoff und Z für Wasserstoff stehen, eine Verbindung der allgemeinen Formel II

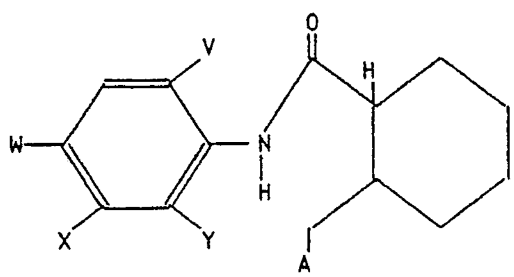

(II) ,

in der V, W, X und Y die unter der allgemeinen Formel I angegebenen Be deutungen haben und A für ein Halogenatom oder einen p-Toluolsulfonyloxyrest steht, gegebenenfalls unter Zugabe einer anorganischen oder organischen Base cyclisiert, oder

B) falls T für Schwefel steht, eine Verbindung der allgemeinen Formel I a

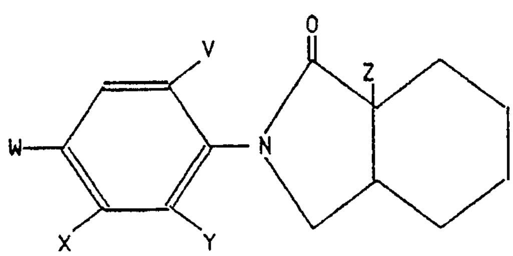

(I a) ,

in der V, W, X, Y und Z die unter der allgemeinen Formel I angegebenen Bedeutungen haben, mit Phosphor(V)-sulfid oder Lawesson's Reagenz umsetzt, oder

C) falls T für Sauerstoff und Z für ein Chlor- oder Bromatom stehen, eine Verbindung der allgemeinen Formel I b

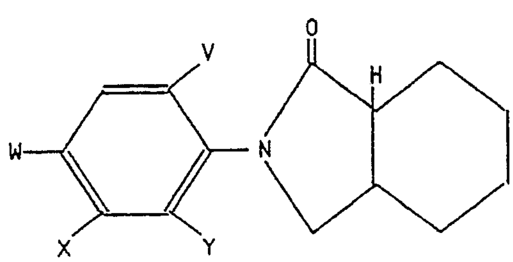

(I b) ,

in der V, W, X und Y die unter der allgemeinen Formel I angegebenen Bedeutungen haben, mit Phosphorhalogeniden und/oder Phosphoroxyhalogeniden umsetzt.

Die Verfahrensvariante A) wird zweckmäßigerweise so durchgeführt, daß man die Ausgangsmaterialien in einem geeigneten Lösungsmittel gegebenenfalls unter Zugabe einer anorganischen oder organischen Base bei Temperaturen zwischen 0°C und 150°C, bevorzugt aber bei Rückflußtemperatur des Lösungsmittels, zur Reaktion bringt. Die Reaktion kann auch gegebenenfalls unter Zugabe eines Phasenstransferkatalysators durchgeführt werden.

Als Basen können Alkali- und Erdalkalihydroxide, Alkoholate, Alkalihydride, Alkali- und Erdalkalicarbonate und -hydrogencarbonate, tertiäre aliphatische und aromatische Amine sowie heterocyclische Basen eingesetzt werden. Beispielhaft seien Natrium- und Kaliumhydroxid, Natriummethanolat, Natriumhydrid, Natrium- und Kaliumcarbonat, Natrium- und Kaliumhydrogencarbonat, Triethylamin und Pyridin genannt.

Als Lösungsmittel kommen Kohlenwasserstoffe, wie zum Beispiel Toluol, chlorierte Kohlenwasserstoffe, wie zum Beispiel Methylenchlorid oder Chloroform, Ether, wie zum Beispiel Diethylether oder Tetrahydrofuran, Alkohole, wie zum Beispiel Methanol oder Ethanol, Ketone, wie zum Beispiel Aceton oder Butanon, Amide, wie zum Beispiel Dimethylformamid, oder auch Sulfoxide, wie zum Beispiel Dimethylsulfoxid, in Frage.

Die zur Cyclisierung benötigten Verbindungen der allgemeinen Formel II werden erhalten, indem man Verbindungen der allgemeinen Formel III

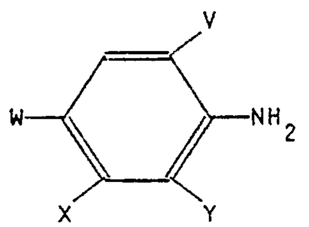

(III) ,

in der V, W, X und Y die in der allgemeinen Formel I angegebenen Bedeutungen haben, mit einer Verbindung der allgemeinen Formel IV

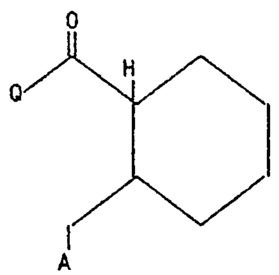

(IV) ,

in der A die unter der allgemeinen Formel II angegebene Bedeutung hat und Q für Chlor oder Brom steht, nach üblichen Methoden umsetzt.

Die Umsetzung gemäß Verfahrensvariante B) wird zweckmäßig bei 0°C bis 150°C in einem geeigneten Lösungsmittel durchgeführt. Als Lösungsmittel kommen Ether, wie zum Beispiel Tetrahydrofuran, aromatische Kohlenwasserstoffe, wie zum Beispiel Toluol, Heteroaromaten, wie zum Beispiel Pyridin, und andere gegenüber den Reaktionspartnern inerte Lösungsmittel in Frage. Gegebenenfalls können der Reaktionslösung auch Basen, wie zum Beispiel Triethylamin, zugesetzt werden. Die Reaktionszeit beträgt 1 bis 24 Stunden.

Die Umsetzung gemäß Verfahrensvariante C) kann gegebenenfalls mit oder ohne Lösungsmittel durchgeführt werden. Die Reaktionstemperaturen liegen zwischen Raumtemperatur und 180°C, bevorzugt aber bei der Rückflußtemperatur des Reaktionsgemisches. Als Lösungsmittel kommen Kohlenwasserstoffe, wie zum Beispiel Benzol, Toluol oder Xylol, oder chlorierte Kohlenwasserstoffe, wie zum Beispiel Methylenchlorid, Chloroform, Chlorbenzol oder Dichlorbenzol, sowie andere gegenüber den Reaktionspartnern inerte Lösungsmittel in Frage. Die Reaktionszeit beträgt 1 bis 24 Stunden.

Die bei den einzelnen Verfahrensvarianten genannten Ausgangsmaterialien sind, sofern die Herstellung nicht beschrieben ist, bekannt oder lassen sich analog zu an sich bekannten Verfahren herstellen.

Die Aufarbeitung der erfindungsgemäßen Verbindungen erfolgt in der üblichen Art und Weise. Eine Aufreinigung erfolgt durch Kristallisation oder Säulenchromatographie.

Die erfindungsgemäßen Verbindungen stellen in der Regel farblose oder schwach gelb gefärbte kristalline oder zähflüssige Substanzen dar, die zum Teil gut löslich in chlorierten Kohlenwasserstoffen, wie zum Beispiel Methylenchorid oder Chloroform, Ethern, wie zum Beispiel Diethylether oder Tetrahydrofuran, Alkoholen, wie zum Beispiel Methanol oder Ethanol, Ketonen, wie zum Beispiel Aceton oder Butanon, Amiden, wie zum Beispiel Dimethylformamid, oder auch Sulfoxiden, wie zum Beispiel Dimethylsulfoxid, sind.

Die erfindungsgemäßen Verbindungen zeigen eine gute herbizide Wirkung bei breitblättrigen Unkräutern und Gräsern. Ein selektiver Einsatz der erfindungsgemäßen Wirkstoffe ist in verschiedenen Kulturen möglich, zum Beispiel in Raps, Rüben Sojabohnen, Baumwolle, Reis, Gerste, Weizen und anderen Getreidearten. Dabei sind einzelne Wirkstoffe als Selektivherbizide in Rüben, Baumwolle, Soja und Getreide besonders geeignet. Ebenso können die Verbindungen zur Unkrautbekämpfung in Dauerkulturen, wie zum Beispiel Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, und zur selektiven Unkrautbekämpfung in einjährigen Kulturen eingesetzt werden.

Die erfindungsgemäßen Verbindungen können zum Beispiel bei den folgenden Pflanzengattungen verwendet werden:

Dikotyle Unkräuter der Gattungen Sinapis, Lepidium, Galium, Stellaria, Matricaria, Anthemis, Galinsoga, Chenopodium, Brassica, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Lamium, Veronica, Abutilon, Datura, Viola, Galeopsis, Papaver, Centaurea und Chrysanthenum.

Monokotyle Unkräuter der Gattungen Avena, Alopecurus, Echinochloa, Setaria, Panicum, Digitaria, Poa, Eleusine, Brachiaria, Lolium, Bromus, Cyperus, Agropyron, Sagittaria, Monochoria, Fimbristylis, Eleocharis, Ischaemum und Apera.

Die Aufwandmengen schwanken je nach Anwendungsart im Vor- und Nachauflauf in Grenzen zwischen 0,001 bis 5 kg/ha.

Die erfindungsgemäßen Wirkstoffe können auch als Defoliant, Desiccant und als Krautabtötungsmittel verwendet werden.

Die erfindungsgemäßen Verbindungen können entweder allein, in Mischung miteinander oder mit anderen Wirkstoffen angewendet werden. Gegebenenfalls können andere Pflanzenschutz- oder Schädlingsbekämpfungsmittel je nach dem gewünschten Zweck zugesetzt werden. Sofern eine Verbreiterung des Wirkungsspektrums beabsichtigt ist, können auch andere Herbizide zugesetzt werden.

Beispielsweise eignen sich als herbizid wirksame Mischungspartner diejenigen Wirkstoffe, die in Weed Abstracts, Vol. 36, No. 12, 1987, unter dem Titel "List of common names and abbreviations employed for currently used herbicides and plant growth regulators in Weed Abstracts" aufgeführt sind.

Eine Förderung der Wirkintensität und der Wirkungsgeschwindigkeit kann zum Beispiel durch wirkungssteigernde Zusätze, wie organische Lösungsmittel, Netzmittel und Öle, erzielt werden. Solche Zusätze lassen daher gegebenenfalls eine Verringerung der Wirkstoffdosierung zu.

Zweckmäßig werden die gekennzeichneten Wirkstoffe oder deren Mischungen in Form von Zubereitungen, wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz-, Emulgier- und/oder Dispergierhilfsmitteln angewandt.

Geeignete flüssige Trägerstoffe sind zum Beispiel aliphatische und aromatische Kohlenwasserstoffe, wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

**A) Spritzpulver**

1.) 20 Gewichtsprozent Wirkstoff
68 Gewichtsprozent Kaolin
10 Gewichtsprozent Calciumsalz der Ligninsulfonsäure
2 Gewichtsprozent Dialkylnaphthalinsulfonat

2.) 40 Gewichtsprozent Wirkstoff
25 Gewichtsprozent Kaolin
25 Gewichtsprozent kolloidale Kieselsäure
8 Gewichtsprozent Calciumsalz der Ligninsulfonsäure
2 Gewichtsprozent Natriumsalz des N-Methyl-N-oleyl-taurins

**B) Paste**

45 Gewichtsprozent Wirkstoff
5 Gewichtsprozent Natriumaluminiumsilikat
15 Gewichtsprozent Cetylpolyglycolether mit 8 Mol Ethylenoxid
2 Gewichtsprozent Spindelöl
10 Gewichtsprozent Polyethylenglycol
23 Gewichtsprozent Wasser

**C) Emulsionskonzentrat**

20 Gewichtsprozent Wirkstoff
75 Gewichtsprozent Isophoron
2 Gewichtsprozent ethoxyliertes Rizinusöl
3 Gewichtsprozent Calciumsalz der Dodecylphenylsulfonsäure

Die folgenden Beispiele erläutern die Herstellung der erfindungsgemäßen Verbindungen:

**Beispiel 1**

**trans-2-[4-Chlor-2-fluor-5-(2-propinyloxy)-phenyl]-perhydro-isoindol-1-on (racemisch)**

8,04 g 2-(Brommethyl)-N-[4-chlor-2-fluor-5-(2-propinyloxy)-phenyl]-cyclohexancarbonsäureamid werden mit 5,52 g Kaliumcarbonat in 200 ml Acetonitril 2 Stunden zum Sieden erhitzt und danach auf Eiswasser gegossen. Die Kristalle werden abgesaugt und aus Diisopropylether/Isopropanol umkristallisiert.
Ausbeute: 6,06 g = 63 % der Theorie
Fp.: 116°C

Das Ausgangsmaterial wird wie folgt hergestellt:

**2-(Brommethyl)-N-[4-chlor-2-fluor-5-(2-propinyloxy)-phenyl]-cyclohexancarbonsäuremamid**

29,94 g 4-Chlor-2-fluor-5-(2-propinyloxy)-anilin und 15,15 g Triethylamin werden in 100 ml Acetonitril gelöst. Danach werden 35,93 g 2-(Brommethyl)-cyclohexancarbonsäurechlorid zugetröpft und 1 Stunde nachgerührt. Nach dem Abkühlen wird auf Wasser gegeben, mit Dichlormethan extrahiert, die organische

Phase mit 5%-iger Salzsäurelösung gewaschen und über Magnesiumsulfat getrocknet. Das nach dem Einengen erhaltene Rohprodukt wird aus Diisopropylether umkristallisiert.
Ausbeute: 25,6 g = 42 % der Theorie
Fp.: 139°C

Analog zu diesem Verfahren werden auch die in der folgenden Tabelle aufgeführten Cyclohexancarbonsäureamide hergestellt:

| V | W | X | Y | A | Physikalische Konstante |
|---|---|---|---|---|---|
| H | H | $CF_3$ | H | Br | Öl |
| F | Cl | $OCH(CH_3)_2$ | H | Br | Fp.: 158°C |
| F | $S\text{-}CONCH_2C{\equiv}CH$ | | H | Br | halbfest |
| F | $OCH_2\text{-}CONCH_2C{\equiv}CH$ | | H | Br | Fp.: 185°C |
| F | Cl | $OCH_2CO_2C_2H_5$ | H | Br | Fp.: 98°C |
| F | Cl | $SCH_3$ | H | Br | Fp.: 65°C |
| F | Cl | $CO_2C_2H_5$ | H | Br | Fp.: 105°C |
| F | F | H | $N(CH_3)_2$ | Br | Öl |

## Beispiel 2

### 2-[4-Chlor-2-fluor-5-(2-propinyloxy)-phenyl]-perhydro-isoindol-1-thion

2 g 2-[4-Chlor-2-fluor-5-(2-propinyloxy)-phenyl]-perhydro-isoindol-1-on in 20 ml Dimethoxyethan werden mit 2,78 g Lawesson's Reagenz versetzt und 15 Stunden bei Raumtemperatur gerührt. Nach dem Einengen wird das Rohprodukt durch Säulenchromatographie an Kieselgel (Elutionsmittel: Hexan/Essigester) gereinigt.
Ausbeute: 1,87 g = 90 % der Theorie
Fp.: 143°C

## Beispiel 3

### 7a-Chlor-2-[4-Chlor-2-fluor-5-(2-propinyloxy)-phenyl]-perhydro-isoindol-1-on

1 g 2-[4-Chlor-2-fluor-5-(2-isopropoxy)-phenyl]-perhydro-isoindol-1-on wird mit 3 g Phosphoroxychlorid und 1,75 g Phosphorpentachlorid 5 Stunden zum Sieden erhitzt. Danach wird im Vakuum eingeengt und das Rohprodukt durch Säulenchromatographie an Kieselgel gereinigt (Elutionsmittel: Hexan).
Ausbeute: 0,4 g - 36 % der Theorie
$n_D^{20}$ : 1,55052

Analog zu diesen Verfahren werden auch die in der folgenden Tabelle aufgeführten Perhydro-isoindole der allgemeinen Formel I erhalten:

| Beispiel | T | V | W | X | Y | Z | Physikalische Konstante |
|---|---|---|---|---|---|---|---|
| 4 | O | H | H | $CF_3$ | H | H | Fp.: 168°C |
| 5 | O | F | Cl | $OCH(CH_3)_2$ | H | H | Fp.: 82°C |
| 6 | O | F | $S\text{-}CONCH_2C{\equiv}CH$ | | H | H | Fp.: 169°C (trans-Isomeres) |
| 7 | O | F | $OCH_2\text{-}CONCH_2C{\equiv}CH$ | | H | H | Fp.: 173°C |
| 8 | O | F | Cl | $OCH_2CO_2C_2H_5$ | H | H | Fp.: 89°C |
| 9 | O | F | Cl | $SCH_3$ | H | H | Fp.: 93°C |
| 10 | O | F | Cl | $CO_2C_2H_5$ | H | H | Fp.: 147°C |
| 11 | O | F | F | H | $N(CH_3)_2$ | H | $n_D^{20}$ : 1,53614 |
| 12 | O | F | Cl | $OCH_2C{\equiv}CH$ | H | H | Fp.: 116°C $[\alpha]_D$: +51,1° (trans-Isomeres) |
| 13 | O | F | Cl | $OCH_2C{\equiv}Ch$ | H | H | Fp.: 116°C |
| 14 | O | F | $S\text{-}CONCH_2C{\equiv}CH$ | | H | H | Fp.: 137°C (cis-Isomeres) |

Die folgenden Beispiele erläutern die Anwendungsmöglichkeiten der erfindungsgemäßen Verbindungen:

**Beispiel A**

Im Gewächshaus wurden die aufgeführten Pflanzenspezies vor dem Auflaufen mit den aufgeführten Verbindungen in einer Aufwandmenge von 0,3 kg Wirkstoff/ha behandelt. Die Verbindungen wurden zu diesem Zweck als Emulsionen oder Suspensionen mit 500 Litern Wasser/ha gleichmäßig über den Boden versprüht. Hier zeigten drei Wochen nach der Behandlung die erfindungsgemäßen Verbindungen eine hohe Kulturpflanzenselektivität in Sojabohne, Baumwolle und Mais bei ausgezeichneter Wirkung gegen das Unkraut. Das Vergleichsmittel zeigte nicht die gleichhohe Aktivität.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung

GLXMA = Glycine maxima
GOSHI = Gossypium hirsutum
ZEAMX = Zea mays
SORHA = Sorghum halepense
ABUTH = Abutilon theophrasti
MATCH = Matricaria chamomilla
POLSS = Polygonum sp.
SOLSS = Solanum sp.
VERPE = Veronica persica
VIOSS = Viola sp.

| Erfindungsgemäße Verbindung | GLXMA | GOSHI | ZEAMX | SORHA | ABUTH | MATCH | POLSS | SOLSS | VERPE | VIOSS |
|---|---|---|---|---|---|---|---|---|---|---|
| Beispiel 1 | 0 | 0 | 0 | 4 | 4 | 4 | 4 | 4 | 4 | 4 |
| Beispiel 2 | 1 | 0 | 0 | 2 | 4 | 4 | 4 | 4 | 4 | 4 |
| Beispiel 5 | 0 | 0 | 0 | 3 | 1 | 4 | 2 | 4 | 0 | - |
| Unbehandelt | 0 | 0 | 0 | 0 | 0 | 0 | 0 | 0 | 0 | 0 |
| Vergleichsmittel | | | | | | | | | | |
| Oxadiazon | 0 | 0 | 0 | 3 | 2 | 3 | 3 | 3 | 1 | 3 |

**Beispiel B**

Im Gewächshaus wurden die aufgeführten Pflanzenspezies nach dem Auflaufen mit den aufgeführten Verbindungen in einer Aufwandmenge von 0,03 kg Wirkstoff/ha behandelt. Die Verbindungen wurden zu diesem Zweck als Emulsionen oder Suspensionen mit 500 Litern Wasser/ha gleichmäßig über die Pflanzen versprüht. Hier zeigt zwei Wochen nach der Behandlung die erfindungsgemäße Verbindung eine hohe Kulturpflanzenselektivität in Weizen und Mais bei ausgezeichneter Wirkung gegen das Unkraut. Das Vergleichsmittel zeigte nicht die gleichhohe Aktivität.

In der folgenden Tabelle bedeuten:

0 = keine Schädigung
1 = 1 - 24 % Schädigung
2 = 25 - 74 % Schädigung
3 = 75 - 89 % Schädigung
4 = 90 - 100 % Schädigung
TRZAX = Triticum aestivum
ZEAMX - Zea mays
PANSS = Panicum maximum
GALAP = Galium aparine
POLSS = Polygonum sp.
SEBEX = Sesbania exaltata
VERPE = Veronica persica

| Erfindungsgemäße Verbindung | TRZAX | ZEAMX | PANSS | GALAP | POLSS | SEBEX | VERPE |
|---|---|---|---|---|---|---|---|
| Beispiel 1 | 1 | 1 | 3 | 3 | 4 | 3 | 3 |
| Unbehandelt | 0 | 0 | 0 | 0 | 0 | 0 | 3 |
| Vergleichsmittel | | | | | | | |
| Oxadiazon | 1 | 1 | 2 | 2 | 2 | 2 | 2 |

**Beispiel C**

Im Gewächshaus wurde die in der Tabelle aufgeführten Verbindungen mit der ebenfalls erwähnten Aufwandmenge appliziert. Heirzu wurden die Wirkstoffe in Form von Zubereitungen auf die Wasseroberfläche in Gefäße mit 1500 ml Wasser pipettiert. Die Testpflanzenarten wurden im 1- bis 3-Blatt-Stadium eingesetzt. Drei Wochen nach der Applikation wurde die Schädigung der Pflanzen bonitiert. Die erfindungsgemäßen Verbindungen zeigten eine starke Wirkung gegen wichtige Reisunkräuter bei gleichzeitiger Selektivität zu Wasserreis. Die Vergleichsmittel zeigten nicht die gleichhohe Wirksamkeit.

In der folgenden Tabelle bedeuten:

0 = keine Schädigung
1 = schwache Schädigung
2 = mittlere Schädigung
3 = starke Schädigung
4 = total vernichtet
- = nicht geprüft

ORYSA = Oryza sativa
ECHCG = Echinochloa crus-galli
CYPDI = Cyperus difformis
SCPJU = Scirpus juncoides
MOOVA = Monochoria vaginalis
CYPSE = Cyperus serotinus
PASDS = Paspalum distichum

| Erfindungsgemäße Verbindungen | Wasserapplikation kg Wirkstoff/ha | ORYSA | ECHCG | CYPDI | SCPJU | MOOVA | CYPSE | PASDS |
|---|---|---|---|---|---|---|---|---|
| Beispiel 1 | 0,05 | 0 | 4 | - | 3 | 4 | 3 | - |
| Beispiel 6 | 0,05 | 0 | 3 | 4 | 3 | 4 | 4 | 3 |
| Beispiel 7 | 0,05 | 0 | 4 | 4 | 4 | 4 | 4 | 4 |
| Beispiel 13 | 0,10 | 0 | 3 | 4 | 3 | 4 | 4 | 2 |
| Beispiel 14 | 0,05 | 0 | - | 4 | - | - | - | - |
| Vergleichsmittel | | | | | | | | |
| Pretilachlor | 0,5 | 0 | 2 | 4 | 3 | 4 | 0 | 0 |
| Mefenacet | 1,0 | 0 | 2 | 3 | 3 | - | - | - |

## Ansprüche

1. 2-Phenyl-perhydro-isoindol-1-one und 1-thione der allgemeinen Formel I

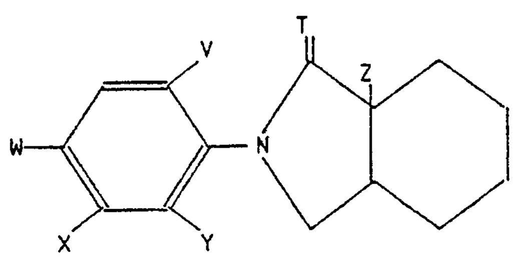

(I) ,

in der
V ein Wasserstoffatom, ein Fluoratom oder ein Chloratom,
W ein Wasserstoffatom oder ein Halogenatom,
X ein Wasserstoffatom, ein Halogenatom, einen Trihalogenmethylrest oder eine der Gruppen $-OR^1$, $-SR^1$ oder $-CO_2R^2$, oder
W und X zusammen eine Gruppierung $-O-CH_2-CO-NR^3-$, $-S-CO-NR^3-$ oder $-O-CO-NR^3-$, wobei $-O-CH_2-$, -S- oder -O- für W stehen,
$R^1$ ein Wasserstoffatom, einen $C_1$-$C_6$-Alkylrest, einen ein- oder mehrfach durch Halogen substituierten $C_1$-$C_6$-Alkylrest, einen $C_2$-$C_6$-Alkenylrest, einen ein- oder mehrfach durch Halogen substituierten $C_2$-$C_6$-Alkenylrest, einen $C_3$-$C_6$-Alkinylrest, einen ein- oder mehrfach durch Halogen substituierten $C_3$-$C_6$-Alkinylrest, einen $C_3$-$C_6$-Cycloalkylrest, einen ein- oder mehrfach durch Halogen substituierten $C_3$-$C_6$-Cycloalkylrest, einen $C_3$-$C_6$-Cycloalkyl-$C_1$-$C_3$-alkylrest, einen ein- oder mehrfach durch Halogen substituierten $C_3$-$C_6$-Cycloalkyl-$C_1$-$C_3$-alkylrest, einen Hydroxycarbonyl-$C_1$-$C_6$-alkylrest, einen ein- oder mehrfach durch Halogen substituierten Hydroxycarbonyl-$C_1$-$C_6$-alkylrest, einen $C_1$-$C_6$-Alkoxycarbonyl-$C_1$-$C_6$-alkylrest, einen ein- oder mehrfach durch Halogen substituierten $C_1$-$C_6$-Alkoxycarbonyl-$C_1$-$C_6$-alkylrest, einen $C_2$-$C_6$-Alkenyloxycarbonyl-$C_1$-$C_6$-alkylrest, einen ein- oder mehrfach durch Halogen substituierten $C_2$-$C_6$-Alkenyloxycarbonyl-$C_1$-$C_6$-alkylrest, einen $C_3$-$C_6$-Alkinyloxycarbonyl-$C_1$-$C_6$-alkylrest, einen ein- oder mehrfach durch Halogen substituierten $C_3$-$C_6$-Alkinyloxycarbonyl-$C_1$-$C_6$-alkylrest, einen $C_3$-$C_6$-

Cycloalkyloxycarbonyl-$C_1$-$C_6$-alkylrest, einen ein- oder mehrfach durch Halogen substituierten $C_3$-$C_6$-Cycloalkyloxycarbonyl-$C_1$-$C_6$-alkylrest, einen $C_1$-$C_4$-Alkylsulfonylrest, einen ein- oder mehrfach durch Halogen substituierten $C_1$-$C_4$-Alkylsulfonylrest, einen Phenylsulfonylrest, einen ein- oder mehrfach, gleich oder verschieden durch Halogen, $C_1$-$C_4$-Alkyl, Halogen-$C_1$-$C_4$-alkyl, Nitro, $C_1$-$C_4$-Alkoxy oder Halogen-$C_1$-$C_4$-alkoxy substituierten Phenylsulfonylrest, einen durch einen gesättigten oder ungesättigten Heterocyclus substituierten $C_1$-$C_4$-Alkylrest, einen Phenylrest oder einen ein- oder mehrfach, gleich oder verschieden durch Halogen, $C_1$-$C_4$-Alkyl, Halogen-$C_1$-$C_4$-alkyl, Nitro, $C_1$-$C_4$-Alkoxy oder Halogen-$C_1$-$C_4$-alkoxy substituierten Phenylrest,

$R^2$ ein Wasserstoffatom, einen $C_1$-$C_6$-Alkylrest, einen ein- oder mehrfach durch Halogen substituierten $C_1$-$C_6$-Alkylrest, einen $C_2$-$C_6$-Alkenylrest, einen ein- oder mehrfach durch Halogen substituierten $C_2$-$C_6$-Alkenylrest, einen $C_3$-$C_6$-Alkinylrest, einen ein- oder mehrfach durch Halogen substituierten $C_3$-$C_6$-Alkinylrest, einen $C_3$-$C_6$-Cycloalkylrest, einen ein- oder mehrfach durch Halogen substituierten $C_3$-$C_6$-Cycloalkylrest, einen $C_3$-$C_6$-Cycloalkyl-$C_1$-$C_3$-alkylrest oder einen ein- oder mehrfach durch Halogen substituierten $C_3$-$C_6$-Cycloalkyl-$C_1$-$C_3$-alkylrest,

$R^3$ ein Wasserstoffatom, einen $C_1$-$C_6$-Alkylrest, einen ein- oder mehrfach durch Halogen substituierten $C_1$-$C_6$-Alkylrest, einen $C_2$-$C_6$-Alkenylrest, einen ein- oder mehrfach durch Halogen substituierten $C_2$-$C_6$-Alkenylrest, einen $C_3$-$C_6$-Alkinylrest, einen ein- oder mehrfach durch Halogen substituierten $C_3$-$C_6$-Alkinylrest, einen $C_3$-$C_6$-Cycloalkylrest, einen ein- oder mehrfach durch Halogen substituierten $C_3$-$C_6$-Cycloalkylrest, einen $C_3$-$C_6$-Cycloalkyl-$C_1$-$C_3$-alkylrest oder einen ein- oder mehrfach durch Halogen substituierten $C_3$-$C_6$-Cycloalkyl-$C_1$-$C_3$-alkylrest,

Y ein Wasserstoffatom, ein Fluoratom, ein Chloratom oder eine Gruppe $NR^4R^5$,

$R^4$ ein Wasserstoffatom, einen $C_1$-$C_6$-Alkylrest, einen $C_2$-$C_6$-Alkenylrest oder einen $C_3$-$C_6$-Alkinylrest,

$R^5$ ein Wasserstoffatom, einen $C_1$-$C_6$-Alkylrest, einen $C_2$-$C_6$-Alkenylrest oder einen $C_3$-$C_6$-Alkinylrest,

T ein Sauerstoffatom oder ein Schwefelatom und

Z ein Wasserstoffatom, ein Chloratom oder ein Bromatom,

bedeuten.

2. Verfahren zur Herstellung von Verbindungen der allgemeinen Formel I, dadurch gekennzeichnet, daß man

A) falls T für Sauerstoff und Z für Wasserstoff stehen, eine Verbindung der allgemeinen Formel II

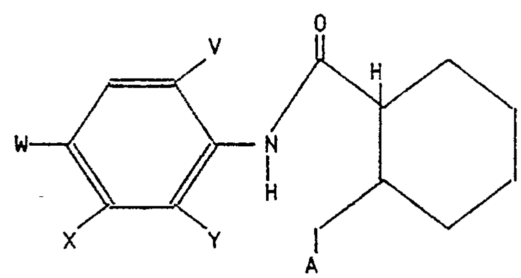

(II) ,

in der V, W, X und Y die unter der allgemeinen Formel I angegebenen Bedeutungen haben und A für ein Halogenatom oder einen p-Toluolsulfonyloxyrest steht, gegebenenfalls unter Zugabe einer anorganischen oder organischen Base cyclisiert, oder

B) falls T für Schwefel steht, eine Verbindung der allgemeinen Formel I a

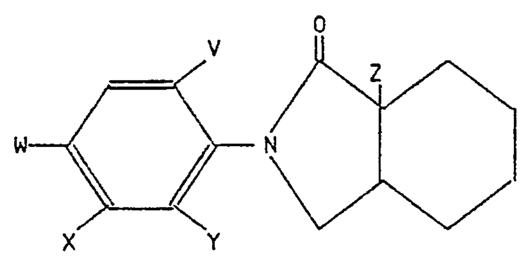

(I a) ,

in der V, W, X, Y und Z die unter der allgemeinen Formel I angegebenen Bedeutungen haben, mit Phosphor(V)-sulfid oder Lawesson's Reagenz umsetzt, oder

C) falls T für Sauerstoff und Z für ein Chlor- oder Bromatom stehen, eine Verbindung der

allgemeinen Formel I b

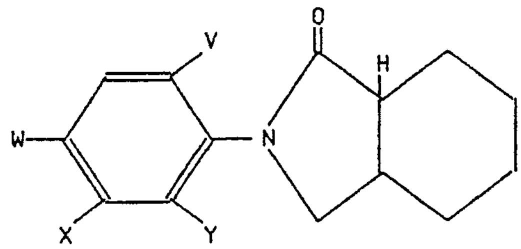

(I b) ,

in der V, W, X und Y die unter der allgemeinen Formel I angegebenen Bedeutungen haben, mit Phosphorhalogeniden und/oder Phosphoroxyhalogeniden umsetzt.

3. Mittel mit herbizider Wirkung, gekennzeichnet durch einen Gehalt an mindestens einer Verbindung gemäß dem Anspruch 1.

4. Verwendung von Mitteln gemäß dem Anspruch 3 zur Bekämpfung monokotyler und dikotyler Unkrautarten in landwirtschaftlichen Hauptkulturen.

5. Verfahren zur Herstellung von Mitteln mit herbizider Wirkung, dadurch gekennzeichnet, daß man Verbindungen der allgemeinen Formel I gemäß dem Anspruch 1 mit Träger- und/oder Hilfsstoffen vermischt.